(19) Europäisches Patentamt
European Patent Office
Office européen des brevets

(11) **EP 1 703 944 B1**

(12) **EUROPEAN PATENT SPECIFICATION**

(45) Date of publication and mention of the grant of the patent:
**08.07.2015 Bulletin 2015/28**

(51) Int Cl.:
***A61N 1/362*** *(2006.01)*

(21) Application number: **04814649.2**

(22) Date of filing: **17.12.2004**

(86) International application number:
**PCT/US2004/042496**

(87) International publication number:
**WO 2005/063333 (14.07.2005 Gazette 2005/28)**

(54) **ADJUSTMENT OF SEQUENTIAL BIVENTRICULAR PACING PARAMETERS**

EINSTELLUNG DER SEQUENTIELLEN BIVENTRIKULÄREN STIMULATIONSPARAMETER

AJUSTEMENT DE PARAMETRES DE STIMULATION BIVENTRICULAIRE SEQUENTIELLE

(84) Designated Contracting States:
**AT BE BG CH CY CZ DE DK EE ES FI FR GB GR HU IE IS IT LI LT LU MC NL PL PT RO SE SI SK TR**

(30) Priority: **19.12.2003 US 742630**

(43) Date of publication of application:
**27.09.2006 Bulletin 2006/39**

(73) Proprietor: **CARDIAC PACEMAKERS, INC.**
**St. Paul, Minnesota 55112-5798 (US)**

(72) Inventors:
• **KRAMER, Andrew, P.**
**Stillwater, Minnesota 55082 (US)**

• **DING, Jiang**
**Maplewood, Minnesota 55109 (US)**

(74) Representative: **Golkowsky, Stefan et al**
**Pfenning, Meinig & Partner GbR**
**Patent- und Rechtsanwälte**
**Joachimstaler Strasse 10-12**
**10719 Berlin (DE)**

(56) References cited:
**US-A- 5 584 867    US-A- 6 144 879**
**US-A1- 2003 083 704    US-A1- 2003 233 131**

**Description**

This invention pertains to cardiac rhythm management devices such as pacemakers and other implantable devices for treating cardiac dysfunction.

**Background**

Cardiac rhythm management devices are implantable devices that provide electrical stimulation to selected chambers of the heart in order to treat disorders of cardiac rhythm. A pacemaker, for example, is a cardiac rhythm management device that paces the heart with timed pacing pulses. The most common condition for which pacemakers are used is in the treatment of bradycardia, where the ventricular rate is too slow. Atrio-ventricular conduction defects (i.e., AV block) that are permanent or intermittent and sick sinus syndrome represent the most common causes of bradycardia for which permanent pacing may be indicated. If functioning properly, the pacemaker makes up for the heart's inability to pace itself at an appropriate rhythm in order to meet metabolic demand by enforcing a minimum heart rate and/or artificially restoring AV conduction.

Pacing therapy can also be used in the treatment of heart failure, which refers to a clinical syndrome in which an abnormality of cardiac function causes a below normal cardiac output that can fall below a level adequate to meet the metabolic demand of peripheral tissues. When uncompensated, it usually presents as congestive heart failure due to the accompanying venous and pulmonary congestion. Heart failure can be due to a variety of etiologies with ischemic heart disease being the most common. It has been shown that some heart failure patients suffer from intraventricular and/or interventricular conduction defects (e.g., bundle branch blocks) such that their cardiac outputs can be increased by improving the synchronization of ventricular contractions with electrical stimulation. In order to treat these problems, implantable cardiac devices have been developed that provide appropriately timed electrical stimulation to one or more heart chambers in an attempt to improve the coordination of atrial and/or ventricular contractions, termed cardiac resynchronization therapy (CRT). Ventricular resynchronization is useful in treating heart failure because, although not directly inotropic, resynchronization can result in a more coordinated contraction of the ventricles with improved pumping efficiency and increased cardiac output. Currently, a most common form of CRT applies stimulation pulses to both ventricles, either simultaneously or separated by a specified biventricular offset interval, and after a specified atrio-ventricular delay interval with respect to the detection of an intrinsic atrial contraction. Appropriate specification of these pacing parameters is necessary in order to achieve optimum improvement in cardiac function, and it is this problem with which the present invention is primarily concerned.

A system according to the preamble of claim 1 is known from US 2003/233131.

**Summary**

The present invention relates to a system and method for optimally determining pacing parameters for delivering cardiac resynchronization therapy. The system may include an implantable cardiac rhythm management device and an external programmer in communication therewith or the implantable device alone. In accordance with the invention, the system measures one or more intrinsic conduction parameters from electrogram signals generated during intrinsic beats. Optimum pre-excitation timing parameters may then be determined in accordance with formulas that relate the optimum pre-excitation timing parameter to the measured intrinsic conduction parameters as defined by a set of specified coefficients. The specified coefficients may be pre-derived from a linear regression analysis of clinical population data relating particular values of the measured intrinsic conduction parameters to an optimum value of the pre-excitation timing parameter as determined by concurrent measurement of another parameter reflective of cardiac function. Pre-excitation timing parameters which may be optimally determined in this manner are the biventricular offset interval which separates right and left ventricular paces and the atrio-ventricular delay interval used in atrial-tracking or AV sequential pacing.

**Brief Description of the Drawing**

Fig. 1 is a block diagram of an exemplary cardiac device for practicing the present invention.
Fig. 2 illustrates an exemplary algorithm for calculating the atrio-ventricular delay interval used in atrial-tracking and AV sequential pacing modes.
Fig. 3 illustrates an exemplary algorithm for measuring intrinsic conduction parameters.
Fig. 4 illustrates an exemplary algorithm for determining the location of a left ventricular lead.
Fig. 5 illustrates an exemplary algorithm for measuring the relative intrinsic AV conduction delays at two left ventricular sites.

Fig. 6 illustrates an exemplary algorithm for selecting between two alternative left ventricular pacing sites.
Fig. 7 illustrates an exemplary algorithm for determining an optimal biventricular offset interval.
Fig. 8 illustrates an exemplary algorithm for determining separate atrio-ventricular delays for each ventricle.

## Detailed Description

Applying cardiac resynchronization therapy in the most efficacious manner requires optimal selection of a number of pacing parameters. Described below is a cardiac rhythm management device configurable for delivering resynchronization pacing to the left ventricle (LV) and/or the right ventricle (RV) in order to compensate for ventricular conduction delays and improve the coordination of ventricular contractions. In accordance with the present invention, a number of these parameters may be set or adjusted based upon measurements of intra-cardiac conduction times using the sensing channels of an implanted device. Algorithms for setting these pacing parameters may be implemented in either the programming of an external programmer or in the programming of the implanted device itself or as a printed lookup table procedure. In the former embodiment, the external programmer communicates with the implantable device over a telemetry link and receives either raw electrogram data, markers corresponding to particular sensed events, or measurements of the intervals between particular sensed events as computed by the implantable device. The external programmer may then compute optimal settings for pacing parameters which are either transmitted to the implantable device for immediate reprogramming or presented to a clinician operating the external programmer as recommendations. In another embodiment, the implantable device is programmed to automatically set certain pacing parameters in accordance with information gathered from its sensing channels. Along the pacing parameters which may be set by either of these embodiments are the selection of which heart chambers are to be paced, the atrio-ventricular delay interval, the biventricular offset interval, and selection between alternative LV pacing sites.

### 1. Exemplary device description

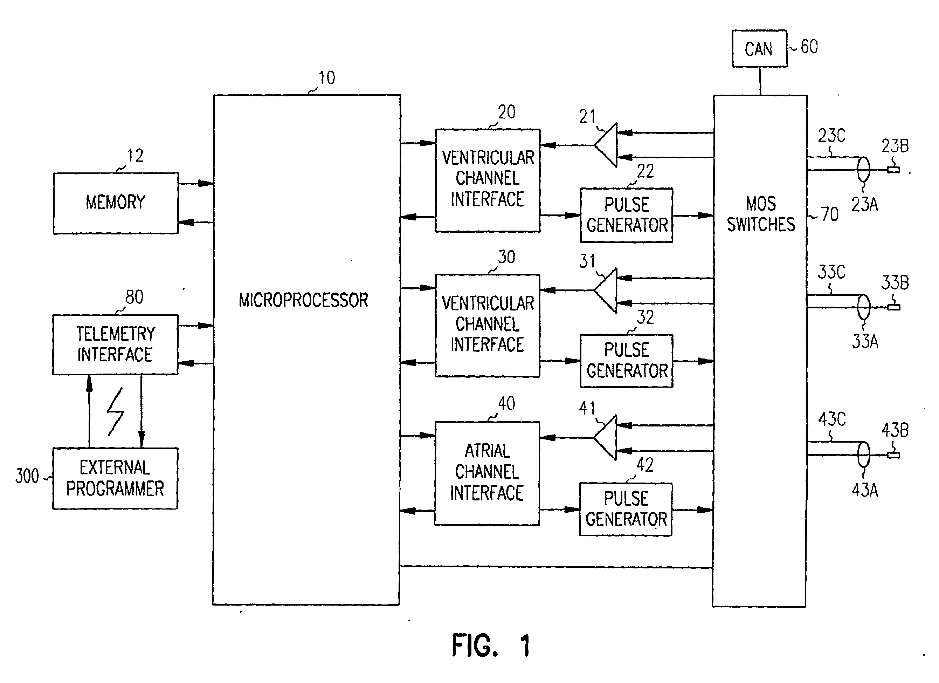

Conventional cardiac pacing with implanted pacemakers involves excitatory electrical stimulation of the heart by the delivery of pacing pulses to an electrode in electrical contact with the myocardium. The pacemaker is usually implanted subcutaneously on the patient's chest, and is connected to electrodes by leads threaded through the vessels of the upper venous system into the heart. An electrode can be incorporated into a sensing channel that generates an electrogram signal representing cardiac electrical activity at the electrode site and/or incorporated into a pacing channel for delivering pacing pulses to the site.
A block diagram of an implantable multi-site pacemaker having multiple sensing and pacing channels is shown in Fig. 1. (As the term is used herein, a "pacemaker" should be taken to mean any cardiac rhythm management device, such as an implantable cardioverter/defibrillator, with a pacing functionality.) The controller of the pacemaker is made up of a microprocessor 10 communicating with a memory 12 via a bidirectional data bus, where the memory 12 typically comprises a ROM (read-only memory) for program storage and a RAM (random-access memory) for data storage. The controller could be implemented by other types of logic circuitry (e.g., discrete components or programmable logic arrays) using a state machine type of design, but a microprocessor-based system is preferable. As used herein, the programming of a controller should be taken to refer to either discrete logic circuitry configured to perform particular functions or to the code executed by a microprocessor. The controller is capable of operating the pacemaker in a number of programmed modes where a programmed mode defines how pacing pulses are output in response to sensed events and expiration of time intervals. A telemetry interface 80 is provided for communicating with an external programmer 300. The external programmer is a computerized device with an associated display and input means that can interrogate the pacemaker and receive stored data as well as directly adjust the operating parameters of the pacemaker. As described below, in certain embodiments of a system for setting pacing parameters, the external programmer may be utilized for computing optimal pacing parameters from data received from the implantable device over the telemetry link which can then be set automatically or presented to a clinician in the form of recommendations.
The embodiment shown in Fig. 1 has three sensing/pacing channels, where a pacing channel is made up of a pulse generator connected to an electrode while a sensing channel is made up of the sense amplifier connected to an electrode. A MOS switching network 70 controlled by the microprocessor is used to switch the electrodes from the input of a sense amplifier to the output of a pulse generator. The switching network 70 also allows the sensing and pacing channels to be configured by the controller with different combinations of the available electrodes. The channels may be configured as either atrial or ventricular channels allowing the device to deliver conventional ventricular single-site pacing with or without atrial tracking, biventricular pacing, or multi-site pacing of a single chamber. In an example configuration, a right atrial sensing/pacing channels includes ring electrode 43a and tip electrode 43b of bipolar lead 43c, sense amplifier 41, pulse generator 42, and a channel interface 40. A right ventricular sensing/pacing channel includes ring electrode 23a and tip electrode 23b of bipolar lead 23c, sense amplifier 21, pulse generator 22, and a channel interface 20, and a left ventricular sensing/pacing channel includes ring electrode 33a and tip electrode 33b of bipolar lead 33c, sense amplifier 31, pulse generator 32, and a channel interface 30. The channel interfaces communicate bi-directionally with a port of microprocessor 10 and include analog-to-digital converters for digitizing sensing signal inputs from the sensing amplifiers, registers that can be written to for adjusting the gain and threshold values of the sensing amplifiers, and registers for controlling the output of pacing pulses and/or changing the pacing pulse amplitude. In this embodiment, the device is equipped with bipolar leads that include two electrodes which are used for outputting a pacing pulse and/or sensing intrinsic activity. Other embodiments may employ unipolar leads with single electrodes for sensing and pacing. The switching network 70 may configure a channel for unipolar sensing or pacing by referencing an electrode of a unipolar or bipolar lead with the device housing or can 60.

The controller 10 controls the overall operation of the device in accordance with programmed instructions stored in memory. The controller 10 interprets electrogram signals from the sensing channels and controls the delivery of paces in accordance with a pacing mode. The sensing circuitry of the pacemaker generates atrial and ventricular electrogram signals from the voltages sensed by the electrodes of a particular channel. An electrogram is analogous to a surface EKG and indicates the time course and amplitude of cardiac depolarization and repolarization that occurs during either an intrinsic or paced beat. When an electrogram signal in an atrial or ventricular sensing channel exceeds a specified threshold, the controller detects an atrial or ventricular sense, respectively, which pacing algorithms may employ to trigger or inhibit pacing.

2. Cardiac resynchronization pacing therapy

Cardiac resynchronization therapy is most conveniently delivered in conjunction with a bradycardia pacing mode. Bradycardia pacing modes refer to pacing algorithms used to pace the atria and/or ventricles in a manner that enforces a certain minimum heart rate. Because of the risk of inducing an arrhythmia with asynchronous pacing, most pacemakers for treating bradycardia are programmed to operate synchronously in a so-called demand mode where sensed cardiac events occurring within a defined interval either trigger or inhibit a pacing pulse. Inhibited demand pacing modes utilize escape intervals to control pacing in accordance with sensed intrinsic activity. In an inhibited demand mode, a pacing pulse is delivered to a heart chamber during a cardiac cycle only after expiration of a defined escape interval during which no intrinsic beat by the chamber is detected. For example, a ventricular escape interval for pacing the ventricles can be defined between ventricular events, referred to as the cardiac cycle (CC) interval with its inverse being the lower rate limit or LRL. The CC interval is restarted with each ventricular sense or pace. In atrial tracking and AV sequential pacing modes, another ventricular escape interval is defined between atrial and ventricular events, referred to as the atrio-ventricular pacing delay interval or AVD, where a ventricular pacing pulse is delivered upon expiration of the atrio-ventricular pacing delay interval if no ventricular sense occurs before. In an atrial tracking mode, the atrioventricular pacing delay interval is triggered by an atrial sense and stopped by a ventricular sense or pace. An atrial escape interval can also be defined for pacing the atria either alone or in addition to pacing the ventricles. In an AV sequential pacing mode, the atrio-ventricular delay interval is triggered by an atrial pace and stopped by a ventricular sense or pace. Atrial tracking and AV sequential pacing are commonly combined so that the AVD starts with either an atrial pace or sense.

As described above, cardiac resynchronization therapy is pacing stimulation applied to one or more heart chambers in a manner that compensates for conduction delays. Ventricular resynchronization pacing is useful in treating heart failure in patients with interventricular or intraventricular conduction defects because, although not directly inotropic, resynchronization results in a more coordinated contraction of the ventricles with improved pumping efficiency and increased cardiac output. Ventricular resynchronization can be achieved in certain patients by pacing at a single unconventional site, such as the left ventricle instead of the right ventricle in patients with left ventricular conduction defects. Resynchronization pacing may also involve biventricular pacing with the paces to right and left ventricles delivered either simultaneously or sequentially, with the interval between the paces termed the biventricular offset (BVO) interval (also sometimes referred to as the LV offset (LVO) interval or VV delay). The offset interval may be zero in order to pace both ventricles simultaneously, or non-zero in order to pace the left and right ventricles sequentially. For one embodiment, the offset interval is signed, with a positive value indicating a pace to the left after the right and a negative value indicating a pace to the left before the right. For another embodiment, the offset interval is the unsigned difference between the atrioventricular pacing delay for the first paced ventricle and the atrioventricular pacing delay for the second paced ventricle.

Cardiac resynchronization therapy is most commonly applied in the treatment of patients with heart failure due to left ventricular dysfunction which is either caused by or contributed to by left ventricular conduction abnormalities. In such patients, the left ventricle or parts of the left ventricle contract later than normal during systole which thereby impairs pumping efficiency. In order to resynchronize ventricular contractions in such patients, pacing therapy is applied such that the left ventricle or a portion of the left ventricle is pre-excited relative to when it would become depolarized in an intrinsic contraction. Optimal pre-excitation in a given patient may be obtained with biventricular pacing or with left ventricular-only pacing.

3. Optimal adjustment of pre-excitation timing parameters

Once a particular resynchronization pacing mode is selected for a patient, pacing parameters affecting the manner and extent to which pre-excitation is applied must be specified. Such pre-excitation timing parameters would include, for example, the atrio-ventricular pacing delay (AVD) and the biventricular offset interval. The biventricular offset interval determines the manner in which the left ventricle is pre-excited relative to right ventricular events. The length of the specified AVD relative to the intrinsic atrio-ventricular delay dictates how early in the cardiac cycle that pacing stimulation is first delivered to the ventricles and, therefore, the amount of pre-excitation delivered to the patient. In order to optimally specify these parameters, the patient may be subjected to clinical hemodynamic testing after implantation where the parameters are varied as cardiac function is assessed. For example, a patient may be given resynchronization stimulation while varying pre-excitation timing parameters in order to determine the values of the parameters that result in maximum cardiac performance, as determined by measuring a parameter reflective of cardiac function such as maximum left ventricular pressure change (dP/dt).

Determining optimal pacing parameters for an individual patient by clinical hemodynamic testing, however, is difficult and costly. It would be advantageous if such optimal pacing parameters could be determined from measurements of intrinsic conduction parameters which reflect how excitation is conducted within the patient's heart during intrinsic beats. In the approach of the present invention, therefore, intrinsic conduction data is collected from a surface EKG or from the sensing channels of the implantable cardiac resynchronization device and then used to compute optimum values of resynchronization pacing parameters. The technique for setting pacing parameters may be implemented as a system in which an external programmer presents intrinsic conduction data to a clinician who then manually programs the implantable device with parameters computed from the intrinsic conduction data (by, for example, using a printed lookup table and procedure). The technique may also be implemented as an automated system for setting optimal pacing parameters. The automated system may be made up of the implantable device alone or an external programmer in communication with the implantable device via a wireless telemetry link. The system may either automatically set the pacing parameters of the implantable device to the computed optimum values or present the optimum values to a clinician in the form of a recommendation. In one embodiment, one or more intrinsic conduction parameters is measured from electrogram signals generated by the sensing channels of an implantable cardiac resynchronization device during intrinsic beats, where the measured intrinsic conduction parameters may represent averages of values obtained during a specified number of intrinsic beats. The automated system, or a clinician manually programming the device, then computes a pre-excitation timing parameter such as the AVD or biventricular offset interval in accordance with a formula that equates an optimum value of the pre-excitation timing parameter to a linear sum of the measured intrinsic conduction parameters multiplied by specified coefficients.

In order to pre-derive the specified coefficients for later programming into the system or for use by a clinician, clinical population data is obtained that relates particular values of the measured intrinsic conduction parameters to an optimum value of the pre-excitation timing parameter as determined by concurrent measurement of another parameter reflective of cardiac function (e.g., maximum dP/dt or minimum atrial rate). A linear regression analysis is then performed to derive values of the specified coefficients used in the formula for setting the pre-excitation timing parameter, the specified coefficients thus being regression coefficients.

a. Optimal adjustment of AVD

The AVD interval determines the amount of pre-excitation delivered by resynchronization, and its optimum value in any particular patient depends on the patient's intrinsic atrio-ventricular intervals and the degree of the patient's conduction pathology. The latter is related to the duration of ventricular depolarization during an intrinsic contraction as reflected by the QRS width in a surface electrocardiogram. It has been found empirically that patients can be categorized into two groups according to how well they respond to resynchronization therapy. It has also been found that patients can be identified as being in one group or another based upon their measured QRS widths. Strong responders, who exhibit a high degree of improvement in systolic function with resynchronization pacing, can be identified as patients with a QRS width greater than 150 milliseconds. Weak responders, who exhibit less improvement with resynchronization pacing, can be identified as patients with a QRS width less than or equal to 150 milliseconds. Within each of the two groups, a linear relationship has been found to exist between the optimal AVD interval for resynchronization pacing and the patient's measured intrinsic atrio-ventricular interval (AVI). Thus, in one embodiment, the optimum AVD interval may be determined by the following formulas:

$$AVD = a_1 AVI + a_2 \quad \text{(for QRS > 150 milliseconds)}$$

or

$$AVD = b_1 AVI + b_2 \quad (\text{for QRS} < 150 \text{ milliseconds})$$

where the coefficients $a_1$, $a_2$, $b_1$, and $b_2$ are obtained from a regression analysis of representative population data. The intrinsic AV interval and QRS width can be determined either from a surface EKG or an intra-cardiac electrogram.

Categorizing patients into only two groups based upon QRS width necessarily has a limited sensitivity and specificity. It has further been found, however, that optimal AVD intervals for resynchronization pacing can be based upon a formula which is a continuous function of both QRS width (or other parameter reflective of depolarization duration) and the measured intrinsic AV delay interval. That is:

$$AVD = k_1 AVI + k_3 QRS + k_4$$

A further refinement of the above formula is to use separately measured intrinsic AV delay intervals for the right and left ventricles as could be obtained from the right ventricular and left ventricular sensing channels of an implanted cardiac device:

$$AVD = k_1 AV_R + k_2 AV_L + k_3 QRS + k_4$$

where $AV_R$ is the intrinsic AV interval for the right ventricle and $AV_L$ is the intrinsic AV interval for the left ventricle.

As noted above, intrinsic conduction parameters for computing the optimum AV delay may be obtained from intra-cardiac electrograms generated by the implanted device's sensing channels. The system may therefore be programmed to measure intrinsic conduction parameters from electrogram signals and use them in a formula with coefficients obtained by linear regression as described above to compute the optimum AVD. The system may be programmed to automatically set the programmed AVD interval in accordance with the computed optimum AVD interval or to recommend to an operator of the external programmer that the programmed AVD interval be set to the computed optimum AVD interval. The measured intrinsic conduction parameters may constitute either single measurements or averages of a plurality of measurements.

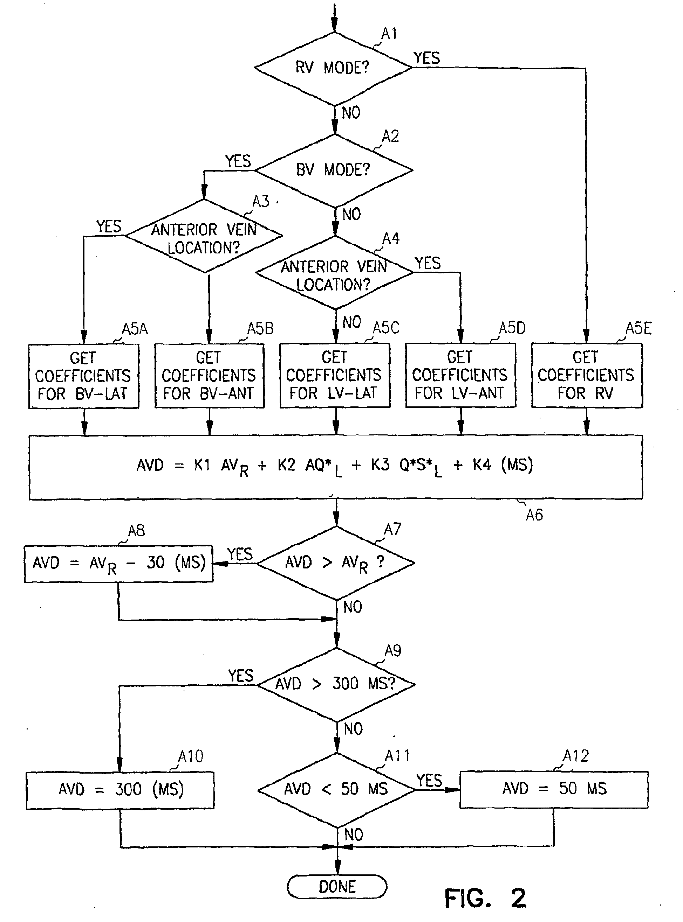

Fig. 2 illustrates an exemplary implementation of the above-described method for determining the optimum AVD as could be executed by an appropriately programmed processor of the implantable cardiac device or external programmer. In this embodiment, the optimum AVD is obtained according to the following formula:

$$AVD = k_1 AV_R + k_2 AQ^*_L + k_3 Q^*S^* + k_4$$

where $AV_R$ is the right intrinsic atrio-ventricular delay measured as the interval between an atrial sense and a right ventricular sense, $AQ^*_L$ is the left intrinsic atrio-ventricular delay measured as the interval between an atrial sense and the start of left ventricular depolarization in an electrogram, and $Q^*S^*$ is the duration of ventricular depolarization measured as the interval from the start of left ventricular depolarization to the end of left ventricular depolarization in an electrogram. The start and end of depolarization may be detected by the implantable device from an electrogram using a threshold criterion similar to that used for detecting chamber senses. The specified coefficients are $k_1$, $k_2$, $k_3$, and $k_4$., where different sets of coefficients are used depending upon which chambers are paced in the currently programmed mode and the location of the left ventricular pacing lead. Each set of specified coefficients may be derived by a regression analysis of clinical data relating the optimum AVD to the measured intrinsic conduction parameters using a pacing mode for pacing a particular chamber or chambers and using a particular location for the left ventricular pacing lead.

Still referring to Fig. 2, the system determines at steps A1 and A2 whether the implantable device is operating in a right ventricular-only pacing mode (RV mode), biventricular pacing mode (BV mode), or left ventricular-only pacing mode (LV mode). If in RV mode, specified coefficients for optimum setting of the AVD interval in that mode are obtained at step A5e. If in BV mode, the system determines at step A3 whether the left ventricular pacing lead is in an anterior or lateral ventricular location, the latter corresponding to the left ventricular free wall. If the left ventricular pacing lead is lateral, specified coefficients for optimum setting of the AVD in that situation are obtained at step A5a, while if the left ventricular pacing lead is anterior, specified coefficients for optimum setting of the AVD are obtained at step A5b. Similarly, if it is determined at step A2 that the device is operating in LV mode, the left ventricular pacing lead location is determined at step A4 so that specified coefficients for setting the AVD are obtained at either step A5c or A5d in accordance with whether the left ventricular pacing lead location is lateral or anterior, respectively. The calculated AVD value is next compared with the right intrinsic atrio-ventricular delay at step A7. If the calculated AVD value is greater than the right intrinsic atrio-ventricular delay, the AVD is set to the value of the latter plus a specified margin (in this case, 30 ms). At steps A9 through A12, the calculated AVD is compared with specified maximum and minimum limit values (300 and 50 ms, respectively, in this case). If the calculated AVD exceeds the maximum limit value, the AVD is set to the maximum limit value. If the calculated AVD is below the minimum limit value, the AVD is set to the minimum limit value. In an alternative embodiment, if the calculated AVD value is greater than the minimum of a fixed percentage of the right intrinsic atrio-ventricular delay (e.g., 70%) or a fixed value (e.g., 300 ms), the AVD is set to the shorter of the two values (i.e., either the fixed percentage of the intrinsic AV delay or the fixed value) or set to a minimum limit value if the calculated AVD is less than the minimum limit value.

Alternative formulas for calculating the AVD using different intrinsic conduction parameters may also be employed, examples of which are:

$$AVD = k_1 AV_R + k_2 AV_L + k_3 Q^*S^* + k_4$$

$$AVD = k_1 AV_R + k_2 Q^*S^* + k_3$$

$$AVD = k_1 AV_L + k_2\ AV_R + k_3$$

$$AVD = k_1 AV_R + k_2 QRS + k_3$$

where $AV_L$ is the left intrinsic atrio-ventricular delay measured as the interval between an atrial sense and a left ventricular sense, and QRS is the width of the QRS complex as determined from a surface electrocardiogram. Which formula would produce the best results in a given situation would depend upon both the individual patient and the particular implanted device. Tables 1 through 3 show example values of specified coefficients for calculating the AVD interval using three of the different formulas discussed above. The coefficients for each formula were calculated by a linear regression analysis of data obtained from a particular clinical population relating the optimum AVD to the intrinsic conduction parameters of the formula. In the tables, the suffix appended to the pacing mode denotes the left ventricular pacing site, either anterior (Ant) or the lateral free wall (Fwl).

**Table 1**

| ($AVD = k1 \cdot AVI_L + k2 \cdot AVI_R + k3$) | | | |
|---|---|---|---|
| | **k1** | **k2** | **k3** |
| **LV-Ant** | 0.163 | 0.769 | -59.6 |
| **BV-Ant** | 0.063 | 1.008 | -73.0 |
| **LV-Fwl** | -0.099 | 0.988 | -64.3 |
| **BV-Fwl** | -0.126 | 0.857 | -27.5 |

**Table 2**

| ($AVD = k1 \cdot QRS + k2 \cdot AVI_R + k3$) | | | |
|---|---|---|---|
| | **k1** | **k2** | **k3** |
| **LV-Ant** | -1.325 | 0.918 | 135.3 |
| **BV-Ant** | -0.835 | 1.041 | 49.0 |
| **LV-Fwl** | -0.459 | 0.911 | -4.3 |
| **BV-Fwl** | -0.728 | 0.757 | 71.3 |

**Table 3**

| (AVD = k1•Q*S* + k2•AQ* + k3• AVI$_R$ + k4) | | | | |
|---|---|---|---|---|
| | **k1** | **k2** | **k3** | **k4** |
| **LV-Ant** | -0.677 | 0.808 | 0.273 | 67.5 |
| **BV-Ant** | -0.706 | 0.615 | 0.610 | 47.2 |
| **LV-Fwl** | -0.337 | 0.157 | 0.797 | -0.46 |
| **BV-Fwl** | -0.312 | 0.339 | 0.482 | 31.6 |

The optimum AVD interval may, in certain instances, differ depending upon whether the ventricular pace or paces are delivered after an atrial sense or after an atrial pace (i.e., whether the paces are delivered in accordance with an atrial tracking mode or an AV sequential pacing mode). It may therefore be desirable to compute separate optimum AVD intervals for the two types of pacing which the implantable device may then use depending upon whether the AVD is initiated by an atrial sense or an atrial pace. Thus, with any of the formulas described above, an optimum AVD delay for use following an atrial pace may be computed with an intrinsic AV interval or intervals measured after an atrial pace, while an optimum AVD delay for use following an atrial sense may be computed with an intrinsic AV interval or intervals measured after an intrinsic atrial beat.

b. Optimal adjustment of biventricular offset interval

As described above, the amount of pre-excitation relative to an atrial beat that is provided to one or both ventricles by resynchronization pacing is determined by the AVD interval. Although it is believed that a primary factor in optimizing ventricular resynchronization pacing is optimal selection of the AVD interval, many patients exhibit further improvement in systolic function with optimal selection of a biventricular offset (BVO) interval (also referred to as the LV offset interval). That is, although certain patients may exhibit optimum improvement with either LV-only pacing (i.e., where only the left ventricle is pre-excited with the right ventricle being intrinsically activated via conduction through the AV node) or biventricular pacing with an arbitrarily chosen biventricular offset, others require biventricular offset pacing with an optimized biventricular offset for optimum improvement in systolic function. For this latter group of patients, it has been found that there is a predictive relationship between the intrinsic right-to-left ventricle conduction time and the best biventricular offset. Preferably, the relationship is expressed as a linear formula:

$$BVO = k1 \bullet \Delta_{RL} + k2$$

where BVO is the optimal biventricular offset interval, $\Delta_{RL}$ is the measured intrinsic right-to-left ventricle conduction time, and k1 and k2 are specified coefficients obtained empirically. Applying this formula results in an optimized biventricular offset for those patients who require it in order to achieve maximum improvement in systolic function with resynchronization pacing.

The $\Delta_{RL}$ parameter may be obtained from electrogram signals generated by the right and left ventricular sensing channels of the implantable device. The $\Delta_{RL}$ parameter may be obtained by the implantable device measuring the time interval between right and left ventricular senses, by the external programmer computing the time interval between RV and LV senses from an electrogram or sense markers transmitted from the implantable device, or by a clinician measuring the distance between RV and LV sense markers in an electrogram display generated by the external programmer. An estimate of the $\Delta_{RL}$ parameter may be measured by other means and used in a linear equation with different coefficients to predict the optimum biventricular offset. Specific estimates of the $\Delta_{RL}$ include the duration of the QRS interval measured on a surface ECG or on a leadless ECG recording from the implanted device, and the Q*S* interval measured from an intracardiac lead electrogram as the interval from the start of left ventricular depolarization to the end of left ventricular depolarization in the electrogram. The $\Delta_{RL}$ parameter may be either a single measurement or an average measurement taken over a number of beats. In one representative patient population, the following formula was found to calculate an optimum biventricular offset interval:

$$BVO = -0.333 \bullet \Delta_{RL} - 20$$

where the specified coefficients were obtained by a regression analysis, and the convention is adopted that a positive $\Delta_{RL}$ value represents the LV sense lagging the RV sense. The system may be programmed to automatically set the programmed biventricular offset interval in accordance with the computed optimum biventricular offset interval or to recommend to an operator of the external programmer that the programmed biventricular offset interval be set to the computed optimum biventricular offset interval.

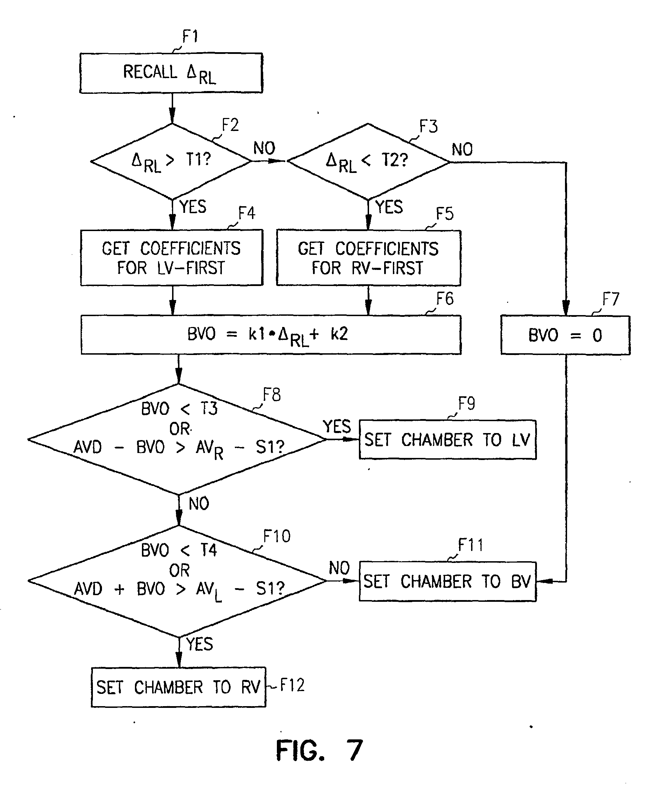

Fig. 7 illustrates as steps F1 through F12 an exemplary implementation of the above-described method for determining the optimum biventricular offset interval as could be executed by an appropriately programmed processor of the implantable cardiac device or external programmer. In this embodiment, the optimum BVO is obtained according to the following formula:

$$BVO = k1 \bullet \Delta_{RL} + k2$$

where $\Delta_{RL}$ is the interval between a right ventricular sense and a left ventricular sense on electrocardiograms. The ventricular sense may be detected by the implantable device from an electrogram using a threshold criterion similar to that used for detecting chamber senses. Alternative formulas for calculating the BVO using different interventricular conduction delay parameters may also be employed, examples of which are:

$$BVO = k1 \bullet QRS + k2$$

$$BVO = k1 \bullet Q^*S^* + k2$$

where QRS is the width width of the QRS complex as determined from a surface electrocardiogram and $Q^*S^*$ is the duration of ventricular depolarization measured as the interval from the start of left ventricular depolarization to the end of left ventricular depolarization in an electrogram. The specified coefficients are k1 and k2, where different sets of coefficients are used depending upon the sign and magnitude of the $\Delta_{RL}$ value. For instance as illustrated in Fig. 7, if $\Delta_{RL}$ is larger than a threshold value, T1, a set of LV-first coefficients is used, otherwise if $\Delta_{RL}$ is less than a threshold value, T2, a set of RV-first coefficients is used. When $\Delta_{RL}$ is greater than T1, there is a right-to-left ventricular conduction delay that can be corrected by pacing the LV first and the RV second; therefore the LV-first coefficients provide a BVO for pacing the LV before the RV. When $\Delta_{RL}$ is less than T2, there is a left-to-right ventricular conduction delay that can be corrected by pacing the RV first and the LV second; therefore the RV-first coefficients provide a BVO for pacing the RV before the LV. Example LV-first coefficients based on population data are k1 = -0.333 and k2 = -20. Example RV-first coefficients based on population data are k1 = 0 and k2 = 0. Also different coefficient sets can be selected based on different locations of the left and right ventricular sensing leads. For example, there can be two LV-first coefficient sets: one to be used when the left ventricular lead is located near the LV septum and another to be used when the left ventricular lead is located near the LV lateral wall. Also different coefficient sets can be selected dependent on whether $\Delta_{RL}$ is measured when atrial sensing or when atrial pacing. When $\Delta_{RL}$ is between T1 and T2, the interventricular conduction delay is too small to be corrected with sequential biventricular pacing; so the BVO is set to zero for simultaneous biventricular pacing. Example threshold values based on population data are T1 = 20 ms and T2 = -20 ms.

Still referring to Fig. 7, after the optimum BVO is calculated, the system selects which chambers are to be paced in delivering ventricular resynchronization. The system determines whether the BVO is smaller than a threshold T3 or if the difference of AVD and BVO is greater than the right intrinsic atrio-ventricular delay ($AV_R$) less an offset S1, and in either case, selects the LV-only chamber for pacing. An example threshold value is T3 = -80 ms. When BVO is less than this value, the conduction from the first LV pace is likely to spread to the right ventricle before it would be paced. Or the RV pace may occur after intrinsic conduction has spread to the right ventricle. This is indicated when the RV pace occurs after an offset S1 from the $AV_R$, where S1 may be for example between 0 and 30 ms. In either case, the effect of the sequential biventricular pacing is equivalent to LV-only pacing. Similarly, the system determines whether the opposite is true, that is whether the BVO is larger than a threshold T4 or if the sum of AVD and BVO is greater than the left intrinsic atrio-ventricular delay ($AV_L$), and in either case, selects the RV-only chamber for pacing. An example threshold value is T4 = 80 ms. If none of these exceptions occur, the system selects the biventricular chambers for pacing with the calculated biventricular offset interval.

3. Exemplary system for determining pre-excitation timing parameters

A system made up of an implantable device and an external programmer (or the implantable device alone) may thus be programmed to determine optimum values for the AVD interval and the biventricular offset interval. In the following description of an exemplary embodiment, the system determines an optimum biventricular offset interval from measurement of the patient's $\Delta_{RL}$ conduction delay. The optimum AVD interval for use with the computed biventricular offset is either determined clinically or determined by the system from a formula utilizing conduction delay alone or combined with QRS width measurements as described above. Most patients with systolic dysfunction which can be improved by resynchronization therapy have conduction deficits which cause delayed intrinsic activation of the left ventricle (e.g., left bundle branch blocks). Systolic function is improved in these patients with resynchronization therapy that pre-excites the left ventricle. The formulas for computing an optimum AVD interval as described above then give an AVD interval representing the time between an atrial sense or pace and a left ventricular pace. The implantable device, however, may deliver ventricular paces in accordance with a pacing mode based upon right ventricular timing. In that case, the AVD interval used by the device is an escape interval started by an atrial sense or pace which is stopped by a right ventricular sense and results in a right ventricular pace upon expiration. In order to reconcile these two types of AVD intervals, the computed optimum biventricular offset is subtracted from the computed optimum AVD interval to give an AVD interval which can then be used by the implantable device with a pacing mode based upon right ventricular timing. For example, if the computed optimum biventricular offset were - 20 ms (i.e., the LV pace leads the RV pace by 20 ms) and the computed optimum AVD interval for left ventricular pre-excitation were 100 ms (i.e., the time between an atrial sense or pace and a left ventricular pace is optimally 100 ms), then the actual AVD delay used by the implantable device using RV-based timing would be 100 - (-20) = 120 ms, which is the time between an atrial sense or pace and a right ventricular pace.

The steps performed by the system in this exemplary embodiment in order to select optimum pre-excitation timing parameters are as follows. First, the intrinsic $\Delta_{RL}$ parameter is determined, either automatically by the implanted device or external programmer or by manually measuring the interval from the implanted device's RV sense to LV sense event markers during intrinsic conduction on the electrogram display. In order to measure the intrinsic $A_{RL}$ interval, the implantable device is set to a temporary sensing diagnostic mode, such as ODO mode. In the case where the system includes an external programmer, transmission of real-time atrial and ventricular markers to the external programmer is also enabled. After the device has transitioned into temporary sensing mode, the implantable device, or an operator of the external programmer, should wait for at least 10 cardiac cycles before measuring the intrinsic $\Delta_{RL}$ interval, and a typical measurement should be made when the intrinsic $\Delta_{RL}$ is stable. Averaging $\Delta_{RL}$ values from several cycles may be helpful. The following events must be avoided when making a measurement: ventricular pacing, intrinsic atrial rate above the programmed maximum tracking rate, premature ventricular contractions, and abnormal atrial or ventricular sensing. The same considerations may also apply when measuring any intrinsic conduction parameter. The intrinsic $\Delta_{RL}$ measurement is then used to calculate the optimum biventricular offset from a formula as described above or used to lookup an optimum biventricular offset from an equivalent table. Once the optimum biventricular offset is determined, an optimum AVD interval is calculated or otherwise obtained. The system may measure intrinsic AV conduction delays and the ventricular depolarization duration in order to compute the optimum AVD interval from a formula as described above. After determination of the optimum AVD interval, the biventricular offset may be subtracted from the optimum AVD interval to give a programmed AVD interval for use in RV-based pacing.

In certain instances, the system may modify the biventricular offset interval from its initially computed optimum value. For example, tachyarrhythmia detection based on right ventricular senses is affected by negative offset pacing of the left ventricle due to a cross-chamber sensing refractory period in the right ventricular sensing channel which is initiated by a left ventricular pace. (See U.S. Patent Application Ser. No. 10/037,444, filed on October 25, 2001, the disclosure of which is hereby incorporated by reference.) That is, even while pacing the heart in a bradycardia resynchronization pacing mode, most cardiac rhythm management devices still monitor intrinsic cardiac activity for the onset of tachyarrhythmias. The device detects a ventricular tachyarrhythmia by measuring the time interval between successive ventricular depolarizations and comparing the measured interval to a specified limit value. That limit value is referred to as the tachyarrhythmia rate threshold interval (TRTI) and corresponds to the lowest intrinsic rate that is to be regarded as a tachyarrhythmia, referred to as the tachyarrhythmia rate threshold (TRT). The effective lower limit for tachyarrhythmia detection corresponds to a maximum tachyarrhythmia rate threshold interval MTRTI that may be expressed in terms of the pacing interval PI and the negative biventricular offset interval BVO as:

$$MTRTI = PI - BVO$$

Thus, decreasing the pacing interval and/or increasing the biventricular offset raises the lowest ventricular rate which can be detected as a tachyarrhythmia. The system may therefore be programmed to compare the maximum pacing rate (e.g., a maximum tracking rate in the case of an atrial tracking pacing mode or a maximum sensor indicated rate in the case of a rate-adaptive pacing mode) and the computed optimum biventricular offset interval to determine if tachyarrhythmia detection would be unduly compromised. The system may then either automatically shorten the biventricular offset interval or display a message advising the clinician to do so. For example, given a particular maximum tracking rate, the system may automatically shorten a computed biventricular offset interval so that the lowest ventricular rate detectable as a tachyarrhythmia is 5 bpm less than the programmed TRT parameter (or advise the clinician to do so via the external programmer).

The system may also incorporate logic for modifying the optimum biventricular offset interval after determination of the more important AVD interval. For example, the system might determine that a zero biventricular offset would be better for the patient. If a zero biventricular offset would be better, the system could either recommend that the biventricular offset be reprogrammed to zero or automatically do so. This may be programmed to occur under any of the following circumstances: 1) positive biventricular offset has been programmed (when a positive biventricular offset should only be programmed as a result of direct hemodynamic testing or other evidence of effectiveness), 2) the system determines the patient is likely to be a weak responder or non-responder to CRT, as indicated by either a QRS < 150 msec or a QRS< 160 msec and the LV lead being in an anterior vein, indicating small baseline asynchrony, which requires a reduced degree of resynchronization that is delivered with longer AV delays resulting in fusion, or 3) the RV activation is delayed compared to the LV activation (i.e., the intrinsic $\Delta_{RL}$ is negative), suggesting a right bundle branch block pattern, which usually means the patient is a weak responder.

4. Optimal adjustment of right and left atrio-ventricular delays for sequential biventricular pacing

Another embodiment of a system for determining pre-excitation timing parameters for cardiac resynchronization therapy determines the biventricular offset interval by selecting two AVD intervals: 1) $AVD_{1ST}$, which is the atrio-ventricular delay to the first paced ventricle, and 2) $AVD_{2ND}$, which is the atrio-ventricular delay to the second paced ventricle. The difference between $AVD_{2ND}$ and $AVD_{1ST}$ is the biventricular offset interval, which is always a positive value. Either the right or left ventricle may be paced first. The optimum AVD intervals are either determined clinically or determined by the system from a formula utilizing conduction delay alone or combined with QRS width measurements as described above. In one case, the optimum AVD intervals are determined for atrial tracking, when the AV intervals are initiated by an atrial sense. In another case, the optimum AVD intervals are determined for AV sequential pacing, when the AV intervals are initiated by an atrial pace. In this embodiment, four optimum AV delay intervals are selected by the system and can be independently programmed into the implantable cardiac device by an external programmer: 1) right sensed AVD, 2) left sensed AVD, 3) right paced AVD, and 4) left paced AVD. When the left ventricle is paced first, left sensed and paced AVD intervals are set to the optimum sensed and paced $AVD_{1ST}$ intervals, and the right sensed and paced AVD intervals are set to the optimum sensed and paced $AVD_{2ND}$ intervals. The opposite assignments are made when the right ventricle is paced first.

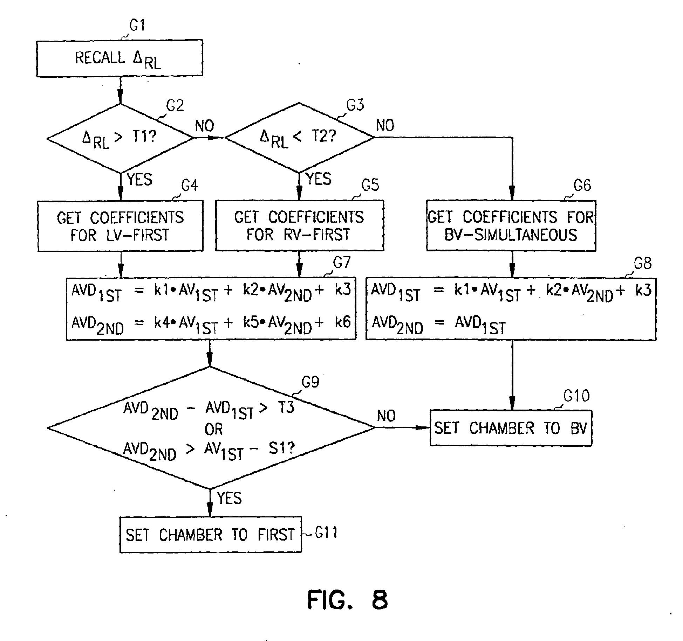

Fig. 8 shows as steps G1 through G11 an exemplary method for determining the optimum $AVD_{1ST}$ and $AVD_{2ND}$ as could be executed by an appropriately programmed processor of the implantable cardiac device or external programmer. In this embodiment, the optimum AVD intervals are obtained according to the following formulas:

$$AVD_{1ST} = k1 \bullet AV_{1ST} + k2 \bullet AV_{2ND} + k3$$

$$AVD_{2ND} = k4 \bullet AV_{1ST} + k5 \bullet AV_{2ND} + k6$$

where $AV_{1ST}$ is the intrinsic interval between an atrial event (sensed or paced) and the right or left ventricular sense whichever is first, and $AV_{2ND}$ is the intrinsic interval between an atrial event and whichever is the second ventricular sense on electrocardiograms. Alternative formulas for calculating the AVD intervals may also be employed as illustrated in the section (Optimal adjustment of AVD) above. In the embodiment in Fig. 8, the coefficient sets (k1,k2,k3) and (k4,k5,k6) for the AVD interval formulas are based upon the sign and magnitude of the $\Delta_{RL}$ value, which is the right-to-left ventricular conduction delay. For instance as illustrated in Fig. 8, if $\Delta_{RL}$ is larger than a threshold value, T1, the left ventricle is selected to be paced first and a set of LV-first coefficients is used, otherwise if $\Delta_{RL}$ is less than a threshold value, T2, the right ventricle is selected to be paced first and a set of RV-first coefficients is used. Example LV-first coefficients for $AVD_{1ST}$ based on population data are presented in Table 1, and example coefficients for $AVD_{2ND}$ are shown in Table 4. Different coefficient sets can be selected based on different locations of the left and right ventricular sensing leads, as illustrated in the tables. Also different coefficient sets can be selected dependent on whether $\Delta_{RL}$ is measured when atrial sensing or when atrial pacing. When $\Delta_{RL}$ is between T1 and T2, the interventricular conduction delay is too small to be corrected with sequential biventricular pacing. In this case, the $AVD_{1ST}$ is calculated based on intrinsic conduction intervals and the $AVD_{2ND}$ is set equal to the $AVD_{1ST}$ for simultaneous biventricular pacing.

**Table 4**

| (AVD$_{2ND}$ = k4•AVI$_L$ + k5• AVI$_R$ + k6) | | |
|---|---|---|
| | **k4** | **k5** | **k6** |
| **LV-Ant** | 0.496 | 0.436 | -39.6 |
| **BV-Ant** | 0.396 | 0.675 | -53.0 |
| **LV-Fwl** | 0.234 | 0.655 | -44.3 |
| **BV-Fwl** | 0.207 | 0.524 | -7.5 |

Still referring to Figure 8, after the optimum AVD intervals are calculated, the system selects which chambers are to be paced in delivering ventricular resynchronization. The system determines whether the biventricular offset interval is larger than a threshold T3 or if the AVD$_{2ND}$ is greater than the right intrinsic atrio-ventricular delay (AV$_R$) less an offset S1. In either case, the first-paced chamber only is selected for pacing. If neither is the case, the system selects the biventricular chambers for pacing with the calculated AVD intervals.

5. Adjustment of other pacing parameters

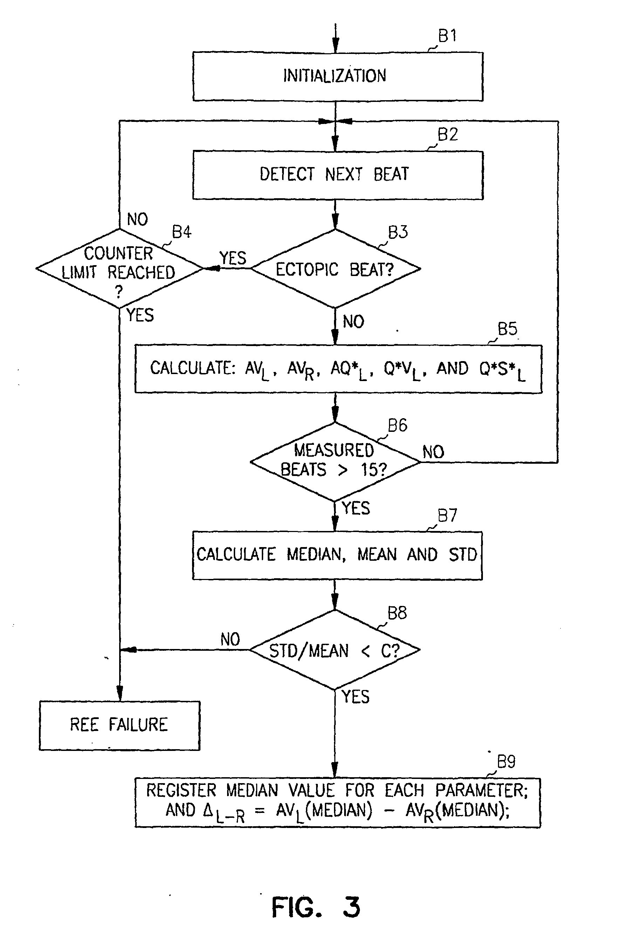

Fig. 3 shows an exemplary algorithm for obtaining the intrinsic conduction parameters used to calculate the pre-excitation timing parameters by the procedures described above as well as select other optimum pacing parameters. (As the terms are used herein, "setting" or "selecting" a particular pacing parameter should be taken to mean either recommending the selected parameter to an operator of the external programmer or automatically configuring the implantable device with the selected parameter.) The algorithm would be executed during a data acquisition period while no pacing therapy is applied by the implantable device. At step B1, the sensing channels for obtaining the RV and LV electrograms are selected, and a beat counter variable is initialized. When an atrial sense occurs, a beat is detected at step B2 and the beat counter variable is incremented. If a subsequent ventricular sense does not occur, the beat is discarded as ectopic at step B3, and, if the beat counter variable does not exceed a specified limit value as tested for at step B4, the algorithm awaits the next beat detection at step B2. Otherwise the intrinsic conduction parameters AV$_L$, AV$_R$, AQ*$_L$, Q*V$_L$, and Q*S*$_L$ are measured from the RV and LV electrogram signals generated during the beat and stored at step B5. AV$_L$, AV$_R$, AQ*$_L$, and Q*S*$_L$ are as defined above, and Q*V$_L$ is the measured interval from the start of left ventricular depolarization to a left ventricular sense. At step B6, the beat counter variable is compared with another specified limit value (in this case, fifteen) and, if the limit value has not been reached, the algorithm waits for the next beat. After conduction parameters from fifteen beats have been stored, the mean, median, and standard deviation values of the parameter measurements are calculated at step B7. A step B8, the ratio of the standard deviation to the mean is calculated for all of conduction parameter measurements and compared to a specified limit value C, where in the case of AV$_L$ and AV$_R$, C = 0.25, while in the case of AQ*$_L$, Q*V$_L$, and Q*S*$_L$, C = 0.4. If the calculated ratios are all less than C, an average value of the stored measurement for each conduction parameter is registered and used to represent that conduction parameter at step B9. The average value of the stored measurements used in this embodiment is a median, but other embodiments may employ a mean, standard deviation, or similar statistic. In addition, an interventricular conduction delay parameter $\Delta_{L-R}$ is calculated as the difference between the average value of AV$_L$ and the average value of AV$_R$.

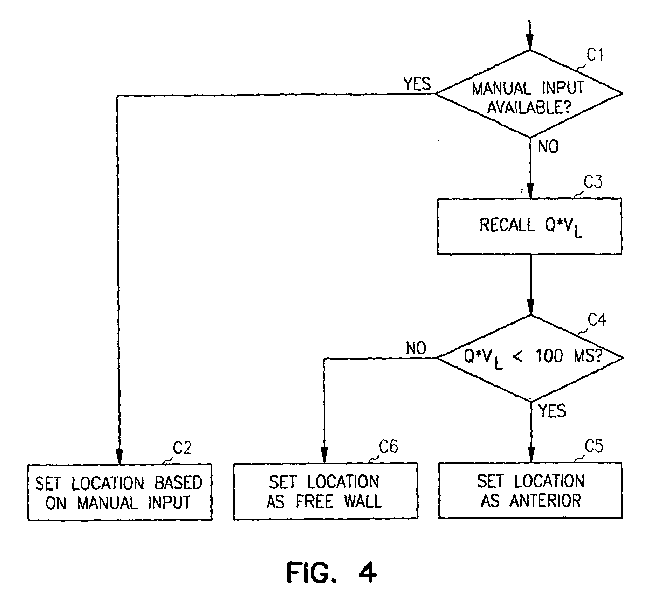

Fig. 4 illustrates an algorithm that may be executed by the system in order to determine the left ventricular lead location, which information is used by the algorithm for calculating the AVD illustrated in Fig. 2 If manual input of the parameter is available, as determined at step C1, the algorithms sets the lead location according to the manual input at step C2. Otherwise, the representative value of the Q*V$_L$ parameter is recalled at step C3 and compared with a limit value (in this case, 100 ms) at step C4. If the Q*V$_L$ parameter is less than the limit value, the lead location is set as anterior at step C5. Otherwise, the lead location is set as the left ventricular free wall at step C6.

The system may also use measured intrinsic conduction parameters to compute other pacing parameters for optimal delivery of therapy. Such parameters may include which heart chambers are to be paced and which alternative LV pacing sites should be used to pace the left ventricle. The left ventricular lead used for sensing and pacing may be a bipolar or multi-polar lead, which thus makes available to the implantable device alternative sites for delivering paces to the left ventricle. The selection between alternative LV pacing sites for optimal delivery of resynchronization therapy can be made based upon the relative intrinsic AV conduction delays measured from the different sites. Under most circumstances, it is desirable to pre-excite the left ventricular region that suffers the most conduction delay during an intrinsic contraction in order to compensate for that delay. For example, if two LV pacing sites L1 and L2 are available, the intrinsic conduction parameters $AV_{L1}$ and $AV_{LV2}$ can be measured which are the intervals between an atrial sense and a left ventricular sense detected from electrograms generated at sites L1 and L2, respectively. The pacing site which is excited later during an intrinsic beat as reflected by a longer AV delay interval can then be selected as the LV pacing site.

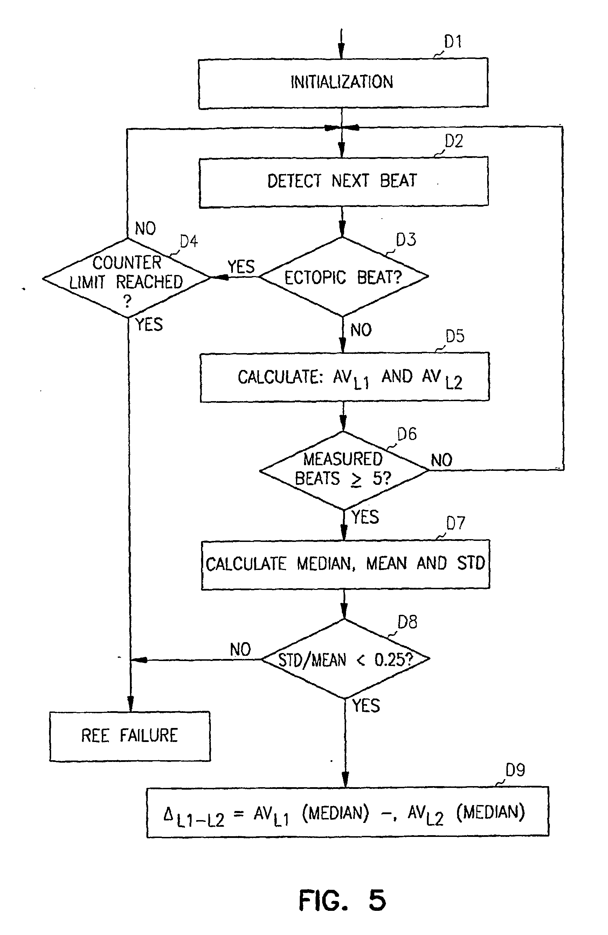

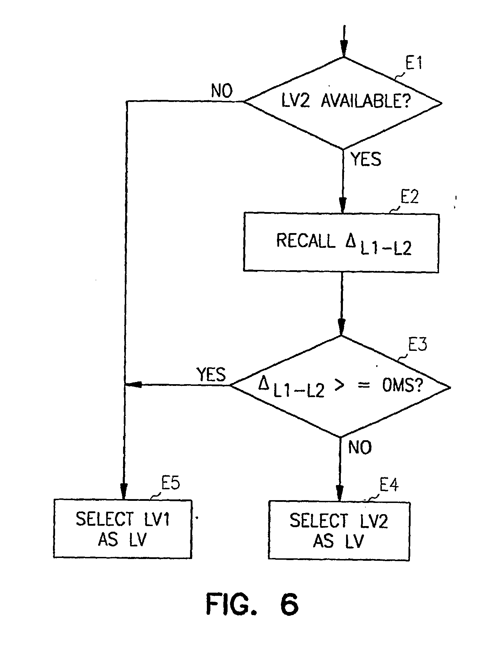

Fig. 5 illustrates an exemplary algorithm for obtaining the $AV_{L1}$ and $AV_{LV2}$ parameters and calculating their difference, designated as $\Delta_{L1-L2}$. At step D1, the beat counter variable is reset, and the implantable device is configured to receive electrograms from alternative sites L1 and L2. In the particular device illustrated in Fig. 1, this would involve configuring two sensing channels with electrodes at the L1 and L2 sites via the switching network 70. Steps D2 through D4 discard ectopic beats and are similar to previously described steps B2 through B4 of Fig. 3. At step D5, the conduction parameters $AV_{L1}$ and $AV_{LV2}$ are measured from the two electrograms. At step D6, the beat counter variable is compared with another specified limit value (in this case, five) and, if the limit value has not been reached, the algorithm waits for the next beat. After measurements of the parameters from five beats have been stored, the mean, median, and standard deviation values of the $AV_{L1}$ and $AV_{LV2}$ measurements are calculated at step D7. At step D8, the ratio of the standard deviation to the mean is calculated and, if found to be less than a specified limit value (in this case, 0.25), $\Delta_{L1-L2}$ is calculated as the difference between the median values of $AV_{L1}$ and $AV_{LV2}$. The system may then be programmed to select the LV pacing site in accordance with the calculated $\Delta_{L1-L2}$ parameter, an exemplary procedure for which is illustrated by Fig. 6. At step E1, the system checks if a pacing site L2 is available and, if not, selects the default site L1 at step E5. If both sites L1 and L2 are available, the $\Delta_{L1-L2}$ parameter is recalled at step E2 and compared with zero at step E3. If $\Delta_{L1-L2}$ is negative, indicating that site L1 is excited earlier than site L2 during an intrinsic contraction, site L2 is selected as the LV pacing site at step E4. If $\Delta_{L1-L2}$ is greater than or equal to zero, indicating that site L1 is excited later than site L2 or at the same time, site L1 is selected as the LV pacing site at step E5.

6. System implementation

The above-described algorithms may thus be used by a system including the implantable device and an external programmer or the implantable device alone in order to set one or more pacing parameters for the optimal delivery of resynchronization therapy. One or more of the algorithms may be executed by the system in order to initialize the parameters prior to delivering resynchronization therapy and/or executed periodically in order to update the parameters. The implantable cardiac rhythm management device would include sensing channels for generating electrogram signals corresponding to electrical activity in an atrium and both the right and left ventricles, right and left ventricular pacing channels for delivering pacing pulses to the right and left ventricles, and a controller for controlling the output of pacing pulses and interpreting electrogram signals, where the controller is programmed to pace at least one ventricle in a manner defined by at least one pre-excitation timing parameter. The system is programmed to measure (or enable measurement of) one or more intrinsic conduction parameters during an intrinsic beat from electrogram signals generated in the sensing channels of the implantable device, including an intrinsic atrio-ventricular delay interval in each ventricular sensing channel, a duration of ventricular depolarization, and a delay between right and left ventricular activation. The system may then be further programmed to select between a biventricular, right ventricular-only, or left ventricular-only pacing mode based upon the measured intrinsic conduction parameters and/or select the value of the pre-excitation timing parameter according to a formula which includes a linear combination of the measured intrinsic conduction parameters as defined by specified coefficients. The pre-excitation timing parameter may be the AVD and/or an offset interval for delivering biventricular or left ventricular-only pacing. The system may be further programmed to select between a biventricular, right ventricular-only, or left ventricular-only pacing mode based upon the measured intrinsic conduction parameters and a specified location of a left ventricular lead used by the left ventricular sensing and pacing channels, where the location of the left ventricular lead is specified by user input or determined from the value of $Q*V_L$. The device may also be equipped with a plurality of sensing/pacing electrodes, sense amplifiers, pulse generators, and a switching network operated by the controller for configuring a sensing channel by connecting a selected electrode pair to a selected sense amplifier and for configuring a pacing channel by connecting a selected electrode pair to a selected pulse generator. The plurality of sensing/pacing electrodes may include at least two alternative left ventricular electrodes located at different left ventricular sites, and the measured intrinsic conduction parameters may include AV conduction delays measured from the at least two alternative left ventricular electrodes. The system may then be programmed to select between the alternative left ventricular electrodes for configuring the left ventricular pacing channel based upon the intrinsic AV conduction delays measured from each alternative left ventricular electrode such as by configuring the left ventricular pacing channel with whichever of the alternative left ventricular electrodes has the longest intrinsic AV conduction delay measured from it.

In one embodiment, the system for selecting optimum pacing parameters includes the implantable device and an external programmer in communication therewith. The processors of the implantable device and of the external programmer are programmed to perform the steps for selecting optimum pacing parameters as described above, where the computational burden may be shared between the two processors in any manner deemed to desirable. The implantable device may collect intrinsic conduction data and transmits the data to the external programmer in various alternative forms. For example, the transmitted intrinsic conduction data may constitute raw electrograms, markers representing particular events and the times of their occurrence, or the derived conduction parameters themselves. Processing of the intrinsic conduction parameters in order to compute optimum pacing parameters by the algorithms described above may then be done entirely by the external programmer or shared between the external programmer and the implantable device. After computation of the optimum pacing parameter values, the external programmer may then automatically program the implantable device with the computed optimum pacing parameter settings or present the optimum values to a clinician operating the external programmer in the form of a recommendation.

Although the invention has been described in conjunction with the foregoing specific embodiments, many alternatives, variations, and modifications will be apparent to those of ordinary skill in the art. Other such alternatives, variations, and modifications are intended to fall within the scope of the following appended claims.

**Claims**

1. A system for setting one of more pacing parameters used by an implantable cardiac rhythm management device in delivering cardiac resynchronization therapy, comprising:

   an implantable cardiac rhythm management device which includes: an atrial sensing channel for generating electrogram signals corresponding to electrical activity in an atrium; right and left ventricular sensing channels for generating electrogram signals corresponding to electrical activity in the right and left ventricles, respectively; right and left ventricular pacing channels for delivering pacing pulses to the right and left ventricles, respectively; a controller for controlling the output of pacing pulses and interpreting electrogram signals, wherein the controller is programmed to deliver a left ventricular pace and a right ventricular pace separated by a programmed biventricular offset interval in accordance with a demand pacing mode; and, a telemetry interface;
   an external programmer with associated display and input means in communication with the implantable device via the telemetry interface; **characterised in that** the system is programmed to generate signals from which the delay between right ventricular and left ventricular activation during an intrinsic beat, referred to as the $\Delta_{RL}$ interval, may be measured; and,
   wherein the system is programmed to compute an optimum biventricular offset interval in accordance with a formula which expresses the optimum biventricular offset interval as a function of the measured $\Delta_{RL}$ interval.

2. The system of claim 1 wherein the system is programmed to measure the $\Delta_{RL}$ interval from electrogram signals received by the implantable device.

3. The system of claim 1 wherein the controller of the implantable device is programmed to pace the ventricles within a programmable atrio-ventricular delay (AVD) interval after an atrial sense in an atrial-tracking mode or an atrial pace in an AV sequential pacing mode.

4. The system of claim 3 wherein the system is programmed to measure at least one intrinsic atrio-ventricular delay interval from electrogram signals during an intrinsic beat and compute an optimum AVD interval in accordance with a formula which expresses the optimum AVD interval as a function of the measured intrinsic atrio-ventricular delay interval.

5. The system of any of claims 1 through 4 wherein the formula for computing the optimum biventricular offset interval BVO is:

$$BVO = k_1(\Delta_{RL}) + k_2$$

   where $k_1$ and $k_2$ are specified coefficients.

6. The system of any of claims 1 through 5 wherein the specified coefficients defining the relationship between the optimum biventricular offset and the measured $\Delta_{RL}$ interval have been pre-derived from a linear regression analysis of clinical population data relating measured $\Delta_{RL}$ intervals to an optimum biventricular offset for delivering cardiac resynchronization therapy as determined by measurement of a cardiac function parameter.

7. The system of claim 5 wherein the formula for computing the optimum biventricular offset interval BVO is:

$$BVO = -.333 \, (\Delta_{RL}) -20.$$

8. The system of any of claims 1 through 7 wherein the system is programmed to automatically set the programmed biventricular offset interval in accordance with the computed optimum biventricular offset interval.

9. The system of any of claims 1 through 8 wherein the system is programmed to recommend to an operator of the external programmer that the programmed biventricular offset interval be set to the computed optimum biventricular offset interval.

10. The system of any of claims 1 through 9 wherein the measured $\Delta_{RL}$ interval represents an average measurement taken over a plurality of intrinsic beats.

11. The system of any of claims 1 through 10 wherein the computed optimum biventricular offset interval is modified in order to maintain a desired tachyarrhythmia rate threshold.

12. The system of any of claims 4 through 11 wherein the system is programmed to measure right and left intrinsic atrio-ventricular delay intervals and a duration of ventricular depolarization from electrogram signals and wherein the formula for computing the optimum AVD interval includes a linear combination of the measured right and left intrinsic atrio-ventricular delays and the measured duration of ventricular depolarization.

13. The system of any of claims 4 through 11 wherein a right intrinsic atrio-ventricular delay is measured as the interval between an atrial sense and a right ventricular sense designated as $AV_R$, a left intrinsic atrio-ventricular delay is measured as the interval between an atrial sense and a left ventricular sense designated as $AV_L$, and the formula for computing the AVD is:

$$AVD = k_1 AV_R + k_2 AV_L + k_3$$

where $k_1$, $k_2$, and $k_3$, are specified coefficients.

14. The system of claim 12 or claim 13 wherein the right intrinsic atrio-ventricular delay is measured as the interval between an atrial sense and a right ventricular sense designated as $AV_R$, the left intrinsic atrio-ventricular delay is measured as the interval between an atrial sense and the start of left ventricular depolarization in an electrogram designated as $AQ^*_L$, the duration of ventricular depolarization is measured as the interval from the start of left ventricular depolarization to the end of left ventricular depolarization in an electrogram designated as $Q^*S^*$, and the formula for computing the optimum AVD is:

$$AVD = k_1 AV_R + k_2 \, AQ^*_L + k_3 Q^*S^* + k_4$$

where $k_1$, $k_2$, $k_3$, and $k_4$ are specified coefficients.

15. The system of any of claims 4 through 14 wherein the intrinsic atrio-ventricular delay is measured as the interval between an atrial sense and a right ventricular sense designated as $AV_R$, a duration of ventricular depolarization is measured as the interval from the start of left ventricular depolarization to the end of left ventricular depolarization in an electrogram designated as $Q^*S^*$, and the formula for computing the optimum AVD is:

$$AVD = k_1 AV_R + k_2 Q^*S^* + k_3$$

where $k_1$, $k_2$, and $k_3$ are specified coefficients.

16. The system of any of claims 4 through 15 wherein specified coefficients defining a linear combination of the measured intrinsic atrio-ventricular delay interval and the measured duration of ventricular depolarization have been pre-derived from a linear regression analysis of clinical population data relating measured intrinsic atrio-ventricular delay intervals and measured durations of ventricular depolarization to an optimum AVD for delivering cardiac resynchronization therapy as determined by measurement of a cardiac function parameter.

17. The system of any of claims 4 through 16 wherein the system is programmed to automatically set the programmed AVD interval in accordance with the computed optimum AVD interval.

18. The system of any of claims 4 through 17 wherein the system is programmed to recommend to an operator of the external programmer that the programmed AVD interval be set to the computed optimum AVD interval.

19. A method for computing an optimum biventricular offset interval for delivering biventricular pacing therapy, wherein the biventricular offset interval is the interval between right and left ventricular paces, comprising:

measuring a delay between right ventricular and left ventricular activation during an intrinsic beat, referred to as the $A_{RL}$ interval; and,
computing an optimum biventricular offset interval in accordance with a formula which expresses the optimum biventricular offset interval as a function of the measured $\Delta_{RL}$ interval.

20. The method of claim 19 wherein the formula for computing the optimum biventricular offset interval BVO is:

$$BVO = k_1(\Delta_{RL}) + k_2$$

where $k_1$ and $k_2$ are specified coefficients defining the relationship between the optimum biventricular offset and the measured $\Delta_{RL}$ interval which have been pre-derived from a linear regression analysis of clinical population data relating measured $\Delta_{RL}$ intervals to an optimum biventricular offset for delivering cardiac resynchronization therapy as determined by measurement of a cardiac function parameter.

**Patentansprüche**

1. System zum Einstellen eines oder mehrerer Schrittgabeparameter, die von einer implantierbaren Herzrhythmus-Managementvorrichtung bei der Zuführung einer Herzresynchronisationstherapie verwendet werden, welches aufweist:

eine implantierbare Herzrhythmus-Managementvorrichtung, welche enthält: einen atrialen Erfassungskanal zum Erzeugen von Elektrogrammsignalen, die der elektrischen Aktivität in einem Vorhof entsprechen; einen rechten und einen linken ventrikulären Erfassungskanal zum Erzeugen von Elektrogrammsignalen, die der elektrischen Aktivität in dem rechten bzw. linken Ventrikel entsprechen; einen rechten und linken ventrikulären Schrittgabekanal zum Liefern von Schrittgabeimpulsen zu dem rechten bzw. linken Ventrikel; eine Steuervorrichtung zum Steuern der Ausgabe von Schrittgabeimpulsen und zum Auswerten von Elektrogrammsignalen, wobei die Steuervorrichtung programmiert ist, eine linke ventrikuläre Schrittgabe und eine rechte ventrikuläre Schrittgabe getrennt durch ein programmiertes biventrikuläres Versetzungsintervall gemäß einem Anforderungsschrittgabemodus zu liefern; und eine Telemetrieschnittstelle;
eine externe Programmiervorrichtung mit assoziierten Anzeige- und Eingabemitteln in Kommunikation mit der implantierbaren Vorrichtung über die Telemetrieschnittstelle;
**dadurch gekennzeichnet, dass**
das System programmiert ist zum Erzeugen von Signalen, von denen die Verzögerung zwischen der rechten ventrikulären und der linken ventrikulären Aktivierung während eines intrinsischen Herzschlags, die als das $\Delta_{RL}$-Intervall bezeichnet wird, gemessen werden kann; und
das System programmiert ist, ein optimales biventrikuläres Versetzungsintervall gemäß einer Formel zu berechnen, die das optimale biventrikuläre Versetzungsintervall als eine Funktion des gemessenen $\Delta_{RL}$-Intervalls ausdrückt.

2. System nach Anspruch 1, bei dem das System programmiert ist, das $\Delta_{RL}$-Intervall anhand von Elektrogrammsignalen, die von der implantierbaren Vorrichtung empfangen wurden, zu messen.

3. System nach Anspruch 1, bei dem die Steuervorrichtung der implantierbaren Vorrichtung programmiert ist für eine Schrittgabe der Ventrikel innerhalb eines programmierbaren atrioventrikulären Verzögerungsintervalls (AVD-Intervalls) nach einer atrialen Erfassung in einem atrialen Verfolgungsmodus oder einer atrialen Schrittgabe in einem sequentiellen AV-Schrittgabemodus.

4. System nach Anspruch 3, bei dem das System programmiert ist zum Messen zumindest eines intrinsische atrioventrikulären Verzögerungsintervalls anhand von Elektrogrammsignalen während eines intrinsischen Herzschlags und zum Berechnen eines optimalen AVD-Intervalls gemäß einer Formel, die das optimale AVD-Intervall als eine Funktion des gemessenen intrinsischen atrioventrikulären Verzögerungsintervalls ausdrückt.

5. System nach einem der Ansprüche 1 bis 4, bei dem die Formel zum Berechnen des optimalen biventrikulären Versetzungsintervalls BVO lautet:

$$BVO = k_1(\Delta_{RL}) + k_2,$$

worin $k_1$ und $k_2$ bestimmte Koeffizienten sind.

6. System nach einem der Ansprüche 1 bis 5, bei dem die bestimmten Koeffizienten, die die Beziehung zwischen der optimalen biventrikulären Versetzung und dem gemessenen $\Delta_{RL}$-Intervall definieren, vorher aus einer linearen Regressionsanalyse von klinischen Bevölkerungsdaten abgeleitet wurden, die gemessene $\Delta_{RL}$-Intervalle in Beziehung zu einer optimalen biventrikulären Versetzung zum Liefern einer Herzsynchronisationstherapie setzen, die durch Messung eines Herzfunktionsparameters bestimmt wurde.

7. System nach Anspruch 5, bei dem die Formel zum Berechnen des optimalen biventrikulären Versetzungsintervalls BVO lautet:

$$BVO = -0{,}333(\Delta_{RL}) - 20.$$

8. System nach einem der Ansprüche 1 bis 7, bei dem das System programmiert ist zum automatischen Setzen des programmierten biventrikulären Versetzungsintervalls gemäß dem berechneten optimalen biventrikulären Versetzungsintervall.

9. System nach einem der Ansprüche 1 bis 8, bei dem das System programmiert ist, einer Bedienungsperson der externen Programmiervorrichtung zu empfehlen, dass das programmierte biventrikuläre Versetzungsintervall gleich dem berechneten optimalen biventrikulären Versetzungsintervall zu setzen.

10. System nach einem der Ansprüche 1 bis 9, bei dem das gemessene $\Delta_{RL}$-Intervall eine Durchschnittsmessung darstellt, die über mehrere intrinsische Herzschläge durchgeführt wurde.

11. System nach einem der Ansprüche 1 bis 10, bei dem das berechnete optimale biventrikuläre Versetzungsintervall modifiziert ist, um einen gewünschten Tachyarrhythmieraten-Schwellenwert aufrechtzuerhalten.

12. System nach einem der Ansprüche 4 bis 11, bei dem das System programmiert ist, rechte und linke intrinsische atrioventrikuläre Verzögerungsintervalle und eine Dauer ventrikulärer Depolarisation anhand von Elektrogrammsignalen zu messen, und bei dem die Formel zum Berechnen des optimalen AVD-Intervalls eine lineare Kombination der gemessenen rechten und linken intrinsischen atrioventrikulären Verzögerung und der gemessenen Dauer der ventrikulären Depolarisation enthält.

13. System nach einem der Ansprüche 4 bis 11, bei dem eine rechte intrinsische atrioventrikuläre Verzögerung als das Intervall zwischen einer atrialen Erfassung und einer rechtsventrikulären Erfassung, die als $AV_R$ bezeichnet wird, gemessen wird, eine linke intrinsische atrioventrikuläre Verzögerung als das Intervall zwischen einer atrialen Erfassungen und einer linksventrikulären Erfassung, die als $AV_L$ bezeichnet wird, gemessen wird und die Formel zum Berechnen der AVD lautet:

$$AVD = k_1 AV_R + k_2 AV_L + k_3,$$

worin $k_1$, $k_2$ und $k_3$ bestimmte Koeffizienten sind.

14. System nach Anspruch 12 oder Anspruch 13, bei dem die rechte intrinsische atrioventrikuläre Verzögerung als das Intervall zwischen einer atrialen Erfassung und einer rechtsventrikulären Erfassung, die als $AV_R$ bezeichnet wird, gemessen wird, die linke intrinsische atrioventrikuläre Verzögerung als das Intervall zwischen einer atrialen Erfassung und dem Beginn der linksventrikulären Depolarisation in einem Elektrogramm, das als $AQ^*_L$ bezeichnet wird, gemessen wird, die Dauer der ventrikulären Depolarisation als das Intervall von dem Beginn der linksventrikulären Depolarisation bis zum Ende der linksventrikulären Depolarisation in einem Elektrogramm, das als $Q^*S^*$ bezeichnet wird, gemessen wird und die Formel zum Berechnen der optimalen AVD lautet:

$$AVD = k_1 AV_R + k_2 AQ^*_L + k_3 Q^*S^* + k_4,$$

worin $k_1$, $k_2$, $k_3$ und $k_4$ bestimmte Koeffizienten sind.

15. System nach einem der Ansprüche 4 bis 14, bei dem die intrinsische atrioventrikuläre Verzögerung als das Intervall zwischen einer atrialen Erfassung und einer rechtsventrikulären Erfassung, das als $AV_R$ bezeichnet wird, gemessen wird, eine Dauer der ventrikulären Depolarisation als das Intervall von dem Beginn der linksventrikulären Depolarisation bis zu dem Ende der linksventrikulären Depolarisation in einem Elektrogramm, das als $Q^*S^*$ bezeichnet wird, gemessen wird und die Formel zum Berechnen der optimalen AVD lautet:

$$AVD = k_1 AV_R + k_2 Q^*S^* + k_3,$$

worin $k_1$, $k_2$ und $k_3$ bestimmte Koeffizienten sind.

16. System nach einem der Ansprüche 4 bis 15, bei dem bestimmte Koeffizienten, die eine lineare Kombination des gemessenen intrinsischen atrioventrikulären Verzögerungsintervalls und der gemessenen Dauer der ventrikulären Depolarisation definieren, vorher anhand einer linearen Regressionsanalyse von klinischen Bevölkerungsdaten abgeleitet wurden, die gemessene intrinsische atrioventrikuläre Verzögerungsintervalle und gemessene Dauern ventrikulärer Depolarisation zu einer optimalen AVD zum Liefern einer Herzsynchronisationstherapie wie durch Messung eines Herzfunktionsparameters bestimmt in Beziehung setzen.

17. System nach einem der Ansprüche 4 bis 16, bei dem das System programmiert ist zum automatischen Setzen des programmierten AVD-Intervalls gemäß dem berechneten optimalen AVD-Intervall.

18. System nach einem der Ansprüche 4 bis 17, bei dem das System programmiert ist, einer Bedienungsperson der externen Programmiervorrichtung zu empfehlen, dass das programmierte AVD-Intervall gleich dem berechneten optimalen AVD-Intervall gesetzt wird.

19. Verfahren zum Berechnen eines optimalen biventrikulären Versetzungsintervalls zum Liefern einer biventrikulären Schrittgabetherapie, wobei das biventrikuläre Versetzungsintervall das Intervall zwischen rechter und linker ventrikulärer Schrittgabe ist, aufweisend:

Messen einer Verzögerung zwischen rechtsventrikulärer und linksventrikulärer Aktivierung während eines intrinsischen Herzschlags, die als das $\Delta_{RL}$-Intervall bezeichnet wird; und
Berechnen eines optimalen biventrikulären Versetzungsintervalls gemäß einer Formel, die das optimale biventrikuläre Versetzungsintervall als eine Funktion des gemessenen $\Delta_{RL}$-Intervalls ausdrückt.

20. Verfahren nach Anspruch 19, bei dem die Formel zum Berechnen des optimalen biventrikulären Versetzungsintervalls BVO lautet:

$$BVO = k_1 (\Delta_{RL}) + k_2,$$

worin $k_1$ und $k_2$ bestimmte Koeffizienten sind, die die Beziehung zwischen der optimalen biventrikulären Versetzung und dem gemessenen $\Delta_{RL}$-Intervall definieren, die vorher anhand einer linearen Regressionsanalyse von klinischen Bevölkerungsdaten abgeleitet wurden, die gemessene $\Delta_{RL}$-Intervalle auf eine optimale biventrikuläre Versetzung zum Liefern einer Herzsynchronisationstherapie, die durch Messung eines Herzfunktionsparameters bestimmt wurde, beziehen.

## Revendications

1.  Système pour régler l'un de plusieurs paramètres de stimulation utilisés par un dispositif de gestion de rythme cardiaque implantable lors de la délivrance d'une thérapie de resynchronisation cardiaque, comprenant :

    un dispositif de gestion de rythme cardiaque implantable qui inclut : un canal de détection d'oreillette pour générer des signaux d'électrocardiogramme correspondant à une activité électrique dans une oreillette ; des canaux de détection de ventricules droit et gauche pour générer des signaux d'électrocardiogramme correspondant respectivement à une activité électrique dans les ventricules droit et gauche ; des canaux de stimulation de ventricules droit et gauche pour respectivement délivrer des impulsions de stimulation aux ventricules droit et gauche ; un contrôleur pour commander la sortie d'impulsions de stimulation et pour interpréter des signaux d'électrocardiogramme, dans lequel le contrôleur est programmé pour délivrer une stimulation de ventricule gauche et une stimulation de ventricule droit séparées par un intervalle de décalage bi-ventriculaire programmé conformément à un mode de stimulation de demande ; et une interface de télémétrie ;
    un programmateur externe muni de moyens d'affichage et d'entrée en communication avec le dispositif implantable via l'interface de télémétrie,
    **caractérisé en ce que** :

    le système est programmé pour générer des signaux à partir desquels le retard entre une activation de ventricule droit et une activation de ventricule gauche pendant un battement intrinsèque, appelé intervalle $\Delta_{RL}$, peut être mesuré ; et dans lequel :

    le système est programmé pour calculer un intervalle de décalage bi-ventriculaire optimum conformément à une formule qui exprime l'intervalle de décalage bi-ventriculaire optimum en fonction de l'intervalle $\Delta_{RL}$ mesuré.

2.  Système selon la revendication 1, dans lequel le système est programmé pour mesurer l'intervalle $\Delta_{RL}$ à partir de signaux d'électrocardiogramme reçus par le dispositif implantable.

3.  Système selon la revendication 1, dans lequel le contrôleur du dispositif implantable est programmé pour stimuler les ventricules à l'intérieur d'un intervalle de retard atrio-ventriculaire programmable (AVD) après une détection d'oreillette dans un mode de suivi d'oreillette ou après une stimulation d'oreillette dans un mode de stimulation séquentielle AV.

4.  Système selon la revendication 3, dans lequel le système est programmé pour mesurer au moins un intervalle de retard atrio-ventriculaire intrinsèque à partir de signaux d'électrocardiogramme pendant un battement intrinsèque et pour calculer un intervalle AVD optimum conformément à une formule qui exprime l'intervalle AVD optimum en fonction de l'intervalle de retard atrio-ventriculaire intrinsèque mesuré.

5.  Système selon l'une quelconque des revendications 1 à 4, dans lequel la formule pour calculer l'intervalle de décalage bi-ventriculaire optimum BVO est :

$$BVO = k_1\,(\Delta_{RL}) + k_2$$

    dans laquelle $k_1$ et $k_2$ sont des coefficients spécifiés.

6.  Système selon l'une quelconque des revendications 1 à 5, dans lequel les coefficients spécifiés qui définissent la relation entre le décalage bi-ventriculaire optimum et l'intervalle $\Delta_{RL}$ mesuré ont été pré-dérivés à partir d'une analyse par régression linéaire de données de population cliniques qui rapportent des intervalles $\Delta_{RL}$ mesurés à un décalage bi-ventriculaire optimum pour délivrer une thérapie de resynchronisation cardiaque comme déterminé au moyen de la mesure d'un paramètre de fonction cardiaque.

7. Système selon la revendication 5, dans lequel la formule pour calculer l'intervalle de décalage bi-ventriculaire optimum BVO est :

$$BVO = -0,333\ (\Delta_{RL}) - 20.$$

8. Système selon l'une quelconque des revendications 1 à 7, dans lequel le système est programmé pour établir automatiquement l'intervalle de décalage bi-ventriculaire programmé conformément à l'intervalle de décalage bi-ventriculaire optimum calculé.

9. Système selon l'une quelconque des revendications 1 à 8, dans lequel le système est programmé pour recommander à un opérateur du programmateur externe que l'intervalle de décalage bi-ventriculaire programmé soit réglé à l'intervalle de décalage bi-ventriculaire optimum calculé.

10. Système selon l'une quelconque des revendications 1 à 9, dans lequel l'intervalle $\Delta_{RL}$ mesuré représente une mesure moyenne prise sur une pluralité de battements intrinsèques.

11. Système selon l'une quelconque des revendications 1 à 10, dans lequel l'intervalle de décalage bi-ventriculaire optimum calculé est modifié afin de maintenir un seuil de fréquence de tachyarythmie souhaité.

12. Système selon l'une quelconque des revendications 4 à 11, dans lequel le système est programmé pour mesurer des intervalles de retard atrio-ventriculaire intrinsèque droit et gauche et une durée de dépolarisation ventriculaire à partir de signaux d'électrocardiogramme et dans lequel la formule pour calculer l'intervalle AVD optimum inclut une combinaison linéaire des retards atrio-ventriculaires intrinsèques droit et gauche mesurés et de la durée mesurée de dépolarisation ventriculaire.

13. Système selon l'une quelconque des revendications 4 à 11, dans lequel un retard atrio-ventriculaire intrinsèque droit est mesuré en tant qu'intervalle entre une détection d'oreillette et une détection de ventricule droit désigné en tant que $AV_R$, un retard atrio-ventriculaire intrinsèque gauche est mesuré en tant qu'intervalle entre une détection d'oreillette et une détection de ventricule gauche désigné en tant que $AV_L$, et la formule pour calculer AVD est :

$$AVD = k_1\ AV_R + k_2\ AV_L + k_3$$

dans laquelle $k_1$, $k_2$ et $k_3$ sont des coefficients spécifiés.

14. Système selon la revendication 12 ou 13, dans lequel le retard atrio-ventriculaire intrinsèque droit est mesuré en tant qu'intervalle entre une détection d'oreillette et une détection de ventricule droit désigné en tant que $AV_R$, le retard atrio-ventriculaire intrinsèque gauche est mesuré en tant qu'intervalle entre une détection d'oreillette et le début d'une dépolarisation ventriculaire gauche dans un électrocardiogramme désigné en tant que $AQ^*_L$, la durée de dépolarisation ventriculaire est mesurée en tant qu'intervalle depuis le début d'une dépolarisation de ventricule gauche jusqu'à la fin d'une dépolarisation de ventricule gauche dans un électrocardiogramme désigné en tant que $Q^*S^*$, et la formule pour calculer l'AVD optimum est :

$$AVD = k_1\ AV_R + k_2\ AQ^*_L + k_3\ Q^*S^* + k_4$$

dans laquelle $k_1$, $k_2$, $k_3$ et $k_4$ sont des coefficients spécifiés.

15. Système selon l'une quelconque des revendications 4 à 14, dans lequel le retard atrio-ventriculaire intrinsèque est mesuré en tant qu'intervalle entre une détection d'oreillette et une détection de ventricule droit désigné en tant que en tant que $AV_R$, une durée de dépolarisation ventriculaire est mesurée en tant qu'intervalle depuis le début d'une dépolarisation de ventricule gauche jusqu'à la fin d'une dépolarisation de ventricule gauche dans un électrocardiogramme désigné en tant que $Q^*S^*$, et la formule pour calculer l'AVD optimum est :

$$AVD = k_1\ AV_R + k_2\ Q^*S^* + k_3$$

dans laquelle $k_1$, $k_2$ et $k_3$ sont des coefficients spécifiés.

16. Système selon l'une quelconque des revendications 4 à 15, dans lequel des coefficients spécifiés définissant une combinaison linéaire de l'intervalle de retard atrio-ventriculaire intrinsèque mesuré et de la durée mesurée de dépolarisation ventriculaire ont été pré-dérivés à partir d'une analyse par régression linéaire de données de population cliniques rapportant des intervalles de retard atrio-ventriculaire intrinsèque mesurés et des durées mesurées de dépolarisation ventriculaire à un AVD optimum pour délivrer une thérapie de resynchronisation cardiaque comme déterminé par une mesure d'un paramètre de fonction cardiaque.

17. Système selon l'une quelconque des revendications 4 à 16, dans lequel le système est programmé pour établir automatiquement l'intervalle AVD programmé conformément à l'intervalle AVD optimum calculé.

18. Système selon l'une quelconque des revendications 4 à 17, dans lequel le système est programmé pour recommander à un opérateur du programmateur externe que l'intervalle AVD programmé soit réglé à l'intervalle AVD optimum calculé.

19. Procédé pour calculer un intervalle de décalage bi-ventriculaire optimum pour délivrer une thérapie de stimulation bi-ventriculaire, dans lequel l'intervalle de décalage bi-ventriculaire est l'intervalle entre des stimulations de ventricules droit et gauche, comprenant :

la mesure d'un retard entre une activation de ventricule droit et une activation de ventricule gauche pendant un battement intrinsèque, appelé intervalle $\Delta_{RL}$ ; et
le calcul d'un intervalle de décalage bi-ventriculaire optimum conformément à une formule qui exprime l'intervalle de décalage bi-ventriculaire optimum en fonction de l'intervalle $\Delta_{RL}$ mesuré.

20. Procédé selon la revendication 19, dans lequel la formule pour calculer l'intervalle de décalage bi-ventriculaire optimum BVO est :

$$BVO = k_1 \, (\Delta_{RL}) + k_2$$

dans laquelle $k_1$ et $k_2$ sont des coefficients spécifiés qui définissent la relation entre le décalage bi-ventriculaire optimum et l'intervalle $\Delta_{RL}$ mesuré et qui ont été pré-dérivés à partir d'une analyse par régression linéaire de données de population cliniques qui rapportent des intervalles $\Delta_{RL}$ mesurés à un décalage bi-ventriculaire optimum pour délivrer une thérapie de resynchronisation cardiaque comme déterminé au moyen de la mesure d'un paramètre de fonction cardiaque.

FIG. 1

FIG. 2

FIG. 3

FIG. 4

**FIG. 5**

FIG. 6

RECALL $\Delta_{RL}$ — F1

$\Delta_{RL} > T1?$ — F2

NO → $\Delta_{RL} < T2?$ — F3 → NO

YES — F4

GET COEFFICIENTS FOR LV–FIRST — F4

YES — F5

GET COEFFICIENTS FOR RV–FIRST — F5

$BVO = k1 \cdot \Delta_{RL} + k2$ — F6

$BVO = 0$ — F7

$BVO < T3$ OR $AVD - BVO > AV_R - S1?$ — F8

YES → SET CHAMBER TO LV — F9

NO

$BVO < T4$ OR $AVD + BVO > AV_L - S1?$ — F10

NO → SET CHAMBER TO BV — F11

YES

SET CHAMBER TO RV — F12

# FIG. 7

28

FIG. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2003233131 A

- US 037444 A